# EUROPEAN PATENT APPLICATION

(11) **EP 1 680 968 A1**
(43) Date of publication of application: **19.07.2006**
(21) Application number: 05380009.0
(22) Date of filing: 14.01.2005
(51) Int. Cl.: A43B 7/28, A61F 5/14, B29C 44/12

(54) **A process for producing plantar ortheses or inner soles taking a footprint while walking and directly molding**

(71) Applicant: Luis Mata, Diego, 09004 Burgos (ES)
(72) Inventor: Luis Mata, Diego, 09004 Burgos (ES)
(74) Representative: Manzano Cantos, Gregorio

(57) **Abstract**

A process for producing plantar orthoses or insoles taking a footprint while walking and directly molding, having the purpose of taking the plantar footprint of the patient while walking and wearing his usual shoe, and the molding of the plantar orthosis directly on the foot of the patient and immediately after having carried out the examination and issuing the diagnosis, and immediately checking if the produced insole fulfills the expectations for which it is carried out, mainly distinguishing three insoles: simple pressure relief compensating or corrective insoles; compensating or corrective insoles for the different altered biomechanics; and composite insoles, including the two previous corrections, and the obtainment of which requires isolating cotton socks; 40-gauge polystyrene socks; a vacuum melting machine; a height-adjustable vacuum cushion; wax pencil for skin, and a sheet of paper.

## Description

### OBJECT OF THE INVENTION

The main feature of the process, method or system of the invention is that the molding of the plantar orthosis is designed by means of obtaining the plantar footprint of the patient while walking and with his usual shoe.

The orthosis or insole is molded by the physician directly on the foot of the patient immediately after having carried out the examination and issued his diagnosis.

It is a suitable and reliable process for producing custom-tailored insoles because it allows the physician to check immediately after they are produced if they fulfill the expectations for which they are carried out.

The diversity of materials available for the invention allows for choosing the ideal qualities of flexibility, hardness, thickness or others according to the condition or deformity the patient has, his age, weight, occupation and other aspects.

It is possible to carry out innumerable subsequent modifications without needing to use special machinery, should these modifications be necessary.

To carry out the molding by means of this process, the physician only needs a minimum amount of material due to the fact that it can be molded and demolded as many times needed.

This process allows a considerable savings of the cost of the insole, both in time and in alternative materials and machining thereof. The cost of the materials normally used for producing orthotics is twice that of those used with the process of the invention.

The time the physician invests in producing the plantar orthosis is less than that used by means of any other known systems, given that the insole only needs the lining to be arranged.

A very light-weight and low-volume insole with the perimeter of the last of the shoe the patient uses is obtained by means of this process.

It is a completely clean process, both for the patient and for the facilities where it is carried out, with very few wastes, does not produce dust and is non-contaminating.

To obtain the insole according to the invention, the following is necessary:
- Insulating cotton socks or bags
- 40-gauge polystyrene socks
- Vacuum melting machine
- Height-adjustable vacuum cushion
- Wax pencil for skin.
- A sheet of paper.

The vacuum machine can be used in other normal uses in orthopedics.

The height-adjustable vacuum cushion allows obtaining the mold in a standing position and with the height of the heel the patient normally uses.

The height-adjustable vacuum cushion can be used as a substitute for phenolic foams, with the large advantage that it can be used as many times as needed without producing wastes. Once the footprint is obtained, the positive footprint thereof is obtained and the orthosis is molded on the latter. Thus, the savings in boxes of phenolic foam is considerable and, therefore, no wastes are produced.

For the final finish of the plantar orthoses produced by means of this process, only a polishing machine is required.

If the physician does not have mechanical resources available for finishing the insole, the cost of sending it to a laboratory is much less than if he had to send plaster or foam molds.

With this process, the physician is sure to obtain the insole according to his criteria and diagnosis, preventing, in the case of obtaining the insole by means of molds made of plaster, foams or any other process, alterations in the molding or milling of the insole when the preparation thereof is the responsibility of a laboratory.

### BACKGROUND OF THE INVENTION

There are multiple systems for producing plantar orthotics or insoles, such as the one contained in European patent number EP 0001961411 of the same inventor, disclosing "AN IMPROVED PROCESS FOR PRODUCING INSOLES FOR CORRECTING FOOT DEFORMITIES AND ITS PRODUCT".

One of the most usual processes is pedography and podograms, with which the plantar footprint is obtained in the standing opposition and in some cases while walking barefoot, and based on which the insole is designed. It is a very useful process for producing insoles, however, since a flat image is obtained without an exact reference of the perimeter of the foot, given that this is traced following the perimeter of the foot and depending on the inclination of the utensil with which it is traced, it may give a larger or smaller surface, the parts subsequently used for producing the insole are difficult to place in the suitable site. It is also materially impossible to calculate the height of these parts at each given point of the foot since the image obtained with pedography is, as mentioned, a flat image. For example, those contemplated in patent WO 01/43638 A1 for a "SOCK FOR DETECTION OF PRESSURE POINTS IN FEET" of Charlotte-Mecklenburg Hospital Authority doing business at CAROLINAS MEDICAL CENTER, can be cited.

Another procedure is phenolic foam which the patient steps on, leaving his impression and which is subsequently filled with plaster to obtain the positive footprint and to mold the insole thereon. This is a system which does not allow making the necessary corrections in most cases, especially when it is necessary to make corrections or even to place the foot in the neutral position. This system only allows obtaining the footprint in the standing position and generally, and especially in the cases of hollow feet, the toes are placed in an inappropriate position. It is not a suitable method, mainly because the patient does not have the knowledge necessary to know how to step on the foams correcting his disorder, and the physician is not able to make the necessary corrections. For example, WO 02/085208 A1 for "METHOD AND APPARATUS FOR MEASURING FOOT GEOMETRY" of AMFIT, INC, can be cited.

The negative mold with plaster allows the physician to take the mold in a neutral position or else make corrections. To obtain the mold, the plaster bandages are needed to obtain the negative footprint to then take the positive footprint and thereon mold the material used for producing the insole.

Other more recent processes are the obtainment of a pressure map by means of instrumented insoles or by means of "scanning" the foot, which enables obtaining three-dimensional images based on which the insole is designed and passing this data in electronic media to a milling machine which, generally working on ethyl vinyl acetate sheets, produces the insole. It is a costly procedure in which the specialist will design the orthosis depending on the data supplied by the pressure platform or the instrumented insoles and subsequently sends them to a shop where there is a milling machine available which will interpret the data. By means of these electronic measuring systems, it is assumed that the measurements obtained will always be the same in the same individual and under equal conditions, however, the recordings obtained in the same person and under equal conditions are practically always different. For example, European patent EP 1 371 300 A1, for "A CONTACTED THREE DIMENSIONAL FOOTSOLE MEASURER", of Golden Dragon Orthotics Limited, can be cited.

### CHARACTERISTIC FIELD OF THE INVENTION

The main feature of the invention is that once the condition of the patient is diagnosed, the insoles are designed by means of obtaining the plantar footprint of the patient while walking and wearing his usual shoe, and the insoles are molded directly on his feet by means of heat moldable materials.

The diversity of materials available for the process of the invention allow choosing the ideal qualities of flexibility, hardness, thickness or other suitable qualities according to the condition or deformity the patient has, his age, weight, occupation, or other details concerning the patient, and the possibility of innumerable subsequent modifications without needing to use special machinery should these be necessary.

It is a suitable and reliable process for producing custom-tailored insoles because it allows the physician to check immediately after they are produced if the orthotic fulfills the requirements for which it is carried out.

This process allows a considerable savings of the cost of the insole, both in time and in alternative materials and machining thereof. The cost of the materials normally used for producing orthotics is twice that of those used with the process of the invention.

A very light-weight and low-volume insole with the perimeter of the last of the shoe the patient uses is obtained by means of this process.

It is a completely clean process, both for the patient and for the facilities where it is carried out, with very few wastes, does not produce dust and is non-contaminating.

To obtain the insole according to the invention, the following is necessary:
- Insulating cotton socks
- 40-gauge polystyrene socks or bags
- Vacuum melting machine
- Height-adjustable vacuum cushion
- Wax pencil for skin
- A sheet of paper

For the final finish, only a polishing machine is required.

If the physician does not have mechanical resources available for finishing the insole, the cost of sending it to a laboratory is much less than if he had to send plaster or foam molds.

With this system, the physician is sure of obtaining the insole according to his criteria and diagnosis, preventing, in the case of obtaining the insole by means of molds made of plaster, foams or any other process, alterations in the molding or milling of the insole when the preparation thereof is the responsibility of a laboratory.

### DESCRIPTION OF THE INVENTION

Within the orthotics to be produced by means of this process and according to the guidelines described in the object and field of the invention, mainly three orthotics will be distinguished:
- Simple pressure relief compensating or corrective insoles for restructuring the support of the affected metatarsals, especially those caused by irregularities in the longitudinal model of the metatarsals, or for relieving pressure in a precise area needing this type of treatment. Molding is not needed for these plantar orthoses, whereby only the following steps one, two, three, four and five of the process will be followed, the last step only for removing the middle sole from the shoe, checking that it did not move when the patient put his shoes on.
- Compensating or corrective insoles for the different altered biomechanics of the lower limbs and/or for foot conditions and deformities.
- Composite insoles, including the two previous corrections.

### Step One

A shop form will be used which will be attached to the insole and sent to the laboratory should the physician not have mechanical resources for finishing the insole, which form also indicates the patient information, the materials used to produce the insole, as well as the lining and other additions should these be necessary. The following is proposed as ideal:

| Clinic No. | Patient No. | Name *(it is not necessary to indicate name)* | Shop Identification *(space* to be *filled* out by the *shop)* |
|---|---|---|---|
| *(Observations if considered necessary,* such as *the diagnosis or any other annotation which subsequently may help* to *orient the physician in testing the insole on the patient when the produced insole is received from the shop).* | | | |
| Insoles: Base middle sole: *(resin no.)* Arch support (resin *no.)* + *(resin no.)* Extensions/relief areas: Nora *(thickness)* mm. Others Lining: (lining no.) Lifts: Right *(thickness)* mm. Left *(thickness)* mm. According to attached drawing. For shoe size Wedges: Right *(thickness)* mm. Left *(thickness)* mm. According to attached drawing. For shoe size Limits: Right *(specify inner, outer or both edges) **Left** (specify inner,* outer *or both edges)* Other specifications: | | | |

### Step Two

Depending on the type of insole be to carried out, draw on the skin of the sole of the patient with a wax pencil for skin the retro-capitulum line and/ or relief areas and/or extensions at the level of the metatarsophalangeal joints, as well as any other point or lesion which is considered necessary for the insole to carry out its task.

### Step Three

To obtain a paper middle sole equal to the middle sole of the shoe of the patient, it is necessary for this shoe to have a heelpiece, to which end the perimeter of the shoe can be drawn and the width of the rand will be discounted, or by means of standard middle soles, searching for the one which is adapted to the shoe. Once this insole is obtained and it is checked that it is exactly the same as the middle sole of the shoe, three equal middle soles are drawn:
* two which will be used for obtaining the footprint of the patient while walking.
* another one for designing the pattern of the orthotic.

These middles soles are introduced inside the shoe and are fixed with an adhesive strip on the two faces so that the middle sole does not move when the patient puts the shoe on.

### Step 4

The patient is asked to walk so that the drawing marked on the bottom sole of the patient in step two is transferred to the paper inner sole.

### Step 5

Remove the middle sole from the shoe, checking that it did not move when the patient put his shoe on.

Place this middle sole and mark the retro-capitulum line on the inner sole which had been cut to design the pattern of the insole *(see point* three). Generally, and unless there is a noticeable difference in the length or morphology of the feet, only one pattern will be drawn.

At this time, depending on the diagnosis, the physician will design the pattern, widening the insole at the level of the inner or outer edge, as much as necessary for it to carry out its function.

### Step 6

Once the pattern is obtained, it is passed to the materials chosen for the construction of the insole.

In the event of compensating or corrective insoles for the different altered biomechanics of the lower limbs and/or conditions and deformities of the feet, and in the case of the composite insoles, the supports will preferably be produced with heat moldable resins not containing pentachlorophenol.

Three types of resins are generally combined
- One which is used as a middle sole, therefore it goes in the lower portion of the insole and must be very thin and have high elasticity. Its necessary features are:
   - It must contain PG glue for any of the linings normally used in producing plantar supports.
   - Excellent retention and recovery of shape.
   - Water resistance, transpiration of the foot not affecting it.
   - High resistance and durability.
   - It does not adhere to the fingers when handled.
   - Excellent bonding with all the resins used for producing orthotics.
   - It can be cut by means of scissors or die-cutting.
   - It allows flattening with conventional machines.
   - Storage under 45°C and avoiding direct sunlight.

### Technical specifications:

| | |
|---|---|
| Thickness | 0.3 mm |
| Softening point | 75°C |
| Adhesion temperature | 100-120°C |
| Molding pressure | 4-5 Kg/cm² |

- Another more resistant and less elastic resin. It is used as a support, either alone or in combination with another more elastic resin
   It must be a thermoplastic resin with advanced technological, elastic and break resistant features. Its features are:
   - Three-minute open time after being reactivated.
   - They incorporate a double weave fabric.
   - They can be cut with scissors or die-cutting.
   - They need a temperature of about 90°-100°C for bonding and about 70°-80°C for their activation.
   - They allow for any type of modification, either complete or by areas, by means of the application of hot air or a thermal plate.
   - They can be flattened with steel cylinders- flattening base.
   - They allow for sanding.
   - They are stored at temperatures of less than 50°C. They require no type of special conservation.
- Another less resistant resin than the previous one but more elastic which, combined with the previous ones, confers the orthotics with excellent elasticity and resistance qualities. They correspond to a range of thermoplastics, thermoplastic resins, which, by combining two layers of different features, bring together a set of very interesting features for producing plantar supports by means of any system, directly molding or in a mold, and especially for the orthotics produced by means of the process of the invention.
   ● Once molded, they have shape retention values exceeding 90%.
   ●In the molding process, once they are reactivated between 60-70°C, it has an open working time of about 3 minutes.
   ●It does not adhere to fingers when handled.
   ●Excellent bonding with the previous resins.
   ●It can be cut by means of scissors or by die-cutting.
   ●It allows for flattening with conventional machines.
   ● Storage under 45°C, avoiding direct sunlight.

### Technical specifications:

| | |
|---|---|
| Thicknesses | 0.8-0.9 mm |
| | 0.9-1.0 mm |
| | 1.0-1.1 mm |
| | 1.2-1.3 mm |
| Reactivation time | 60-70°C |
| Adhesion temperature | 110-120°C |
| Molding pressure | 4-5 Kg/cm² |
| Flexibility/Hardness | depending on thickness, smooth, mid, mid-high |

The last two resins are useful for producing the support of the sole. Depending on the diagnosis, weight, activity, or other details, they are combined and melted together, whereupon the necessary elasticity and torsion qualities for the purpose for which they are designed, are obtained.

To obtain all the qualities of the resins, it is fundamental that said resins are well melted together, without the resins changing their perimeter when heated and so that their thickness, on the contrary, is reduced. This is achieved by means of a vacuum melting machine of the same applicant.

They can be combined with one another for the purpose of achieving the qualities necessary in each case.

When it is necessary to achieve greater resistance, it is generally not necessary to increase the overall thickness of the insole since, if needed, the insole can be reinforced at those points in which it is necessary, or "posted" by means of limits.

The transfer of the pattern to the resins is carried out by drawing it on the resins. Once this has been done, the sheets to be melted are trimmed and are identified on the face in contact with the patient with the record number, type of lining and any other feature considered necessary. The resin which will be used as the insole base will also be identified.

### Step Seven

Molding process. Producing plantar orthoses or insoles by means of the process of the invention allows molding the plantar orthotics in:
- A seated position.
- A weight-bearing standing position.
- A combination of the two forms.

Once they are molded, they enable immediately checking, before adding the extensions, should they have any, and placing the lining, if the insole bothers the patient and if it fulfills its objectives. This is one of the advantageous qualities of the invention.

### Molding in a seated position

1.- The insulating cotton socks are placed on the patient. These are single-use socks.
2.- The end of the aspiration hose of the vacuum melting machine is placed at the level of the back of the foot so that it does not interfere in the molding.
3.- The resins are prepared for the molding by introducing them, wrapped in a 40-gauge polystyrene film, into the vacuum melting machine.
4- When the molding temperature is reached, they are removed from the vacuum melting machine and applied on the foot of the patient, on the insulating cotton sock.
   There is no risk of burning the patient mainly because the temperature of the melted resins does not exceed 70°C, and because the physician carries them in his hands to put them on the foot of the patient.
5.- The vacuum valve is opened and the resins are suitably placed on the foot, they are covered with a 40-gauge polystyrene bag-sock, passing over the end of the aspiration hose, and it is closed with a neoprene strip so that the vacuum molds the resins against the foot of the patient. At this time, the physician can carry out all the corrections he deems necessary.
6.- Before the complete cooling, the polystyrene bag and the insole are removed, and it is checked to see if it has stabilized.
7.- If, for any reason, the insole does not fulfill the expectations of the physician, it can be demolded in the vacuum melting machine and molded again, as many times as needed.
   Likewise, if a small area does not fulfill the expectations, this area can be retouched with an air-gun without needing to completely demold it.
   This is another one of the advantages of the process, since any other process for each practice requires the use of new materials, and this process can be carried out with the same material as many times as needed until obtaining the necessary skill.
8.- At this time and once the insole has completely cooled down, it can be checked before the patient leaves if it complies with the necessary conditions for which it was designed and even if it causes any discomfort for the patient, to that end the patient puts his shoes on and walks with the recently molded insole. The physician can thus be sure of its functionality before sending the insole for its finish. It is insisted that this is one of the essential features of the process.

### Molding in a weight-bearing standing position

To carry out the molding when bearing weight, a height-adjustable vacuum cushion is needed, according to the invention with a connection to the vacuum melting machine.

This cushion consists of an enclosure completely adaptable to any shape which, in order to achieve this adaptability, is internally made up of microspheres and is placed on a support allowing its height adjustment depending on the height of the heel of the normal shoe of the patient.
1.- The patient, with the insulating cotton socks, is seated with his feet resting on the cushion adjusted to the height of the heel the patient normally uses. The feet are placed on the cushion according to the diagnosis and the patient stands up. New corrections can be made again at this time if needed. When it is considered that the supports are correct, a vacuum is performed on the cushion, the weight-bearing footprint of the patient remaining therein.
   Steps 2, 3, 4 and 5 are then repeated for the molding in a seated position:
2.- The patient sits down, the end of the aspiration hose is placed under the insulating cotton sock, at the level of the back of the foot so that it does not interfere in the molding.
3.- The resins are prepared for the molding by introducing them, wrapped in a 40-gauge polystyrene film, into the vacuum melting machine.
4- When the molding temperature is reached, they are removed form the vacuum melting machine and applied on the foot of the patient, on the insulating cotton sock.
   There is no risk of burning the patient mainly because the temperature of the melted resins does not exceed 70°C, and because the physician carries them in his hands to put them on the foot of the patient.
5.- The vacuum valve is opened and the resins are suitably placed on the foot, they are covered with a 40-gauge polystyrene bag-sock, passing over the end of the aspiration hose, and it is closed with a neoprene strip so that the vacuum molds the resins against the foot of the patient.
6.- The patient is placed on the weight-bearing footprint described in point 1.
   Before the complete cooling, the polystyrene bag and the insole are removed, and it is checked to see if it has stabilized.
   As with the molding in the seated position,
7.- if, for any reason, the insole does not fulfill the expectations of the physician, it can be demolded in the vacuum melting machine and molded again, as many times as needed. Likewise, if a small area does not fulfill the expectations, this area can be retouched with an air-gun without needing to completely demold it. This is another one of the advantages of this process, since any other process for each practice requires the use of new materials, and this process can be carried out with the same material as many times as needed until obtaining the necessary skill.
8.- At this time and once the insole has completely cooled down, it can be checked before the patient leaves if it complies with the necessary conditions for which it was designed and even if it causes any discomfort for the patient, to that end the patient puts his shoes on and walks with the recently molded insole. The physician can thus be sure of its functionality before sending the insole for its finish. It is insisted that this is one of the essential features of this process.

### Molding combining the two forms

The first five steps are the same as for the molding in a seated position, i.e.:
1.- The insulating cotton socks are placed on the patient. These are single-use socks.
2.- The end of the aspiration hose of the vacuum melting machine is placed at the level of the back of the foot so that it does not interfere in the molding.
3.- The resins are prepared for the molding by introducing them, wrapped in a 40-gauge polystyrene film, into the vacuum melting machine.
4- When the molding temperature is reached, they are removed form the vacuum melting machine and applied on the foot of the patient, on the insulating cotton sock.
   There is no risk of burning the patient mainly because the temperature of the melted resins does not exceed 70°C, and because the physician carries them in his hands to put them on the foot of the patient.
5.- The vacuum valve is opened and the resins are suitably placed on the foot, they are covered with a 40-gauge polystyrene bag-sock, passing over the end of the aspiration hose, and it is closed with a neoprene strip so that the vacuum molds the resins against the foot of the patient. At this time, the physician can carry out all the corrections he deems necessary.
   The process continues:
6.- The patient stands up on the cushion adjusted to the height of the heel which he usually uses and is kept in this position until the insole has cooled down. In this phase, the vacuum of the height-adjustable cushion is not carried out, the patient simply rests on the cushion until the insoles have cooled down.
   As with the molding in the seated position, the following is repeated:
7.- if, for any reason, the insole does not fulfill the expectations of the physician, it can be demolded in the vacuum melting machine and molded again, as many times as needed. Likewise, if a small area does not fulfill the expectations, this area can be retouched with an air-gun without needing to completely demold it. This is another one of the advantages of this process, since any other process for each practice requires the use of new materials, and this process can be carried out with the same material as many times as needed until obtaining the necessary skill.
8.- At this time and once the insole has completely cooled down, it can be checked before the patient leaves if it complies with the necessary conditions for which it was designed and even if it causes any discomfort for the patient, to that end the patient puts his shoes on and walks with the recently molded insole. The physician can thus be sure of its functionality before sending the insole for its finish. It is insisted that this is one of the essential features of this process.

### Step Eight.

Process for the finishing of the plantar orthoses

If the insole has to incorporate extensions or relief areas at the level of the metatarsophalangeal joints, as well as any other addition, or a relief considered necessary for the insole to carry out its function, including the lining, these references from steps one, two, three and four of this process will be taken, i.e. the indications of the work sheet described in step one and obtained while walking as indicated in steps two, three and four.

For the purposes provided for in the process of the invention, a graphic version of the height-adjustable cushion is provided according to a particular interpretation proposed by the invention.

### IN THE DRAWINGS:

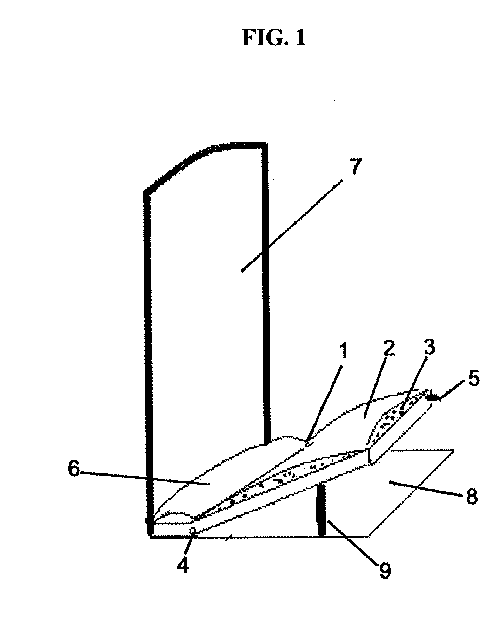

Figure 1 shows a foreshortened side perspective view of the assembly of said height-adjustable vacuum cushion.

### PREFERRED EMBODIMENT OF THE CUSHION

According to the mentioned depiction, the cushion (1) itself is integrated by a cushioned body (2) internally filled with microspheres (3) and externally provided with a valve (5) for producing the internal vacuum. The body (2) is the height-adjustable portion by means of a controlled lifting element (9), assembled on the assembly support platform (8) and functioning at an articulation point (4) with a likewise cushioned fixed portion (6) of the cushion on which a vertical frame or harness (7) is arranged for the patient to hold on to and which, as has been stated, allows obtaining the molding in a standing position and with the height of the heel which the patient normally uses, with the advantage that it can be used as many times as needed, without producing wastes.

Having sufficiently described the features of the invention, it is hereby stated that said invention etc...

## Claims

1. A process for producing plantar orthoses or insoles taking a footprint while walking and directly molding, having as a final purpose taking the plantar footprint of the patient while walking and with his usual shoe, and the molding of the plantar orthoses directly on the foot of the patient and immediately after having performed the examination and issued the diagnosis, and immediately after checking to see if the insole produced fulfills the expectations for which it is carried out, **characterized in that** within the orthotics to be produced by the process, basically three are distinguished:
• Simple pressure relieving compensating or corrective insoles for restructuring the support of the affected metatarsals, especially those caused by irregularities in the longitudinal model of the metatarsals, or for relieving pressure in a precise area needing this type of treatment.
• Compensating or corrective insoles for the different altered biomechanics of the lower limbs and/or for foot conditions and deformities.
• Composite insoles, including the two previous corrections.
And for obtaining them, the following are necessary:
- Insulating cotton socks
- 40-gauge polystyrene socks or bags
- Vacuum melting machine
- Height-adjustable vacuum cushion
- Wax pencil for skin
- A sheet of paper

2. A process for producing plantar orthoses or insoles taking a footprint while walking and directly molding according to the previous claim, for the orthotics to be produced according to the process, **characterized in that** it comprises the following phases:
One: Using a shop form which will be attached to the insole and sent to the laboratory should the physician not have his own mechanical resources, which form also indicates the patient information, the materials used to produce the insole, as well as the lining and other necessary additions.
Two: Depending on the type of insole be to carried out, draw on the skin of the sole of the patient with a wax pencil for skin the retro-capitulum line and/ or relief areas and/or extensions at the level of the metatarsophalangeal joints, as well as any other point or lesion considered necessary.
Three: To obtain a paper middle sole equal to the middle sole of the shoe of the patient, it is necessary for this shoe to have a heelpiece, to which end the perimeter of the shoe can be drawn and the width of the rand will be discounted, or by means of standard middle soles, searching for the one which is adapted to the shoe. Once this insole is obtained and it is checked that it is exactly the same as the middle sole of the shoe, three equal middle soles are drawn:
* two which will be for obtaining the footprint of the patient while walking.
* another one for designing the pattern of the orthotic.
These middles soles are introduced inside the shoe and are fixed with an adhesive strip on both faces so that the middle sole does not move when the patient puts the shoe on.
Four: The patient is asked to walk so that the drawing marked on the bottom sole of the patient in step two is transferred to the paper inner sole.
Five: Remove the middle sole from the shoe, checking that it did not move when the patient put his shoe on.
Six: Once the pattern is obtained, the process is passed to the materials chosen for the construction of the insole.
Seven: Proceed to the molding process.

3. A process for producing plantar orthoses or insoles taking a footprint while walking and directly molding according to claim 2, once the inner sole of phase five is removed, it is **characterized in that** the middle sole is placed and the retro-capitulum line is marked on the inner sole which had been cut to design the pattern of the insole, this insole flaring out at the level of the inner or outer edge as much as necessary.

4. A process for producing plantar orthoses or insoles taking a footprint while walking and directly molding according to claim 2, once the pattern of phase six is obtained, it is **characterized in that** in the case of compensating or corrective insoles, and in the case of composite insoles, the supports are produced with heat moldable resins which do not contain pentachlorophenol, in which three types of resin intervene:
- One which is used as a middle sole at the lower portion of the insole and must be very thin and have high elasticity.
- Two, a more resistant and less elastic resin. It is used as a support, either alone or in combination with another more elastic resin.
- Three, a less resistant resin than the previous one but more elastic which, combined with the previous ones, confers the orthotics with excellent elasticity and resistance qualities.

5. A process for producing plantar orthoses or insoles taking a footprint while walking and directly molding according to claim 4, the insoles produced with heat moldable resins are **characterized in that** the last two resins are used for producing the support of the sole by combining them or melting them to obtain the necessary elasticity and torsion qualities for the purpose for which they are designed, without the resins changing their perimeter when heated and so that their thickness is reduced, which is achieved by means of a vacuum melting machine.

6. A process for producing plantar orthoses or insoles taking a footprint while walking and directly molding according to claim 6, because in order to achieve more resistance in the insole, it is **characterized in that** it is reinforced at those points in which it is necessary by "posting", or by means of limits.

7. A process for producing plantar orthoses or insoles taking a footprint while walking and directly molding according to claim 4, the transfer of the pattern to the resins is **characterized in that** it is carried out by drawing such pattern on the resins, trimming the sheets to be melted, and they are identified on the face in contact with the patient with the record number, type of lining and any other feature, also identifying the resin which will be used as the insole base.

8. A process for producing plantar orthoses or insoles taking a footprint while walking and directly molding according to claim 2, the molding process of phase seven is **characterized in that** it allows molding plantar orthotics in:
- A seated position.
- A weight-bearing standing position.
- A combination of both forms, enabling immediately checking, essentially, if the insole bothers the patient and if it fulfills its objectives.

9. A process for producing plantar orthoses or insoles taking a footprint while walking and directly molding according to claim 8, the molding process in a seated position is **characterized in that**:
- The single-use insulating cotton socks are placed on the patient.
- The end of the aspiration hose of the vacuum melting machine is placed at the level of the back of the foot so that it does not interfere in the molding.
- The resins are prepared for the molding by introducing them, wrapped in a 40-gauge polystyrene film, into the vacuum melting machine.
- When the molding temperature is reached, they are removed form the vacuum melting machine and applied on the foot of the patient, on the insulating cotton sock, at a temperature of less than 70°C.
- The vacuum valve is opened and the resins are suitably placed on the foot, they are covered with a 40-gauge polystyrene bag-sock, passing over the end of the aspiration hose, and it is closed with a neoprene strip so that the vacuum molds the resins against the foot of the patient, carrying out all the corrections deemed necessary.
- Before the complete cooling, the polystyrene bag and the insole are removed, and it is checked to see if it has stabilized.
- The insole can be demolded in the vacuum melting machine and molded again if does not fulfill the expectations of the physician as many times as needed.
- A small area not fulfilling expectations can be retouched with an air-gun without needing to completely demold it.
- Once the insole has completely cooled down, it can be checked before the patient leaves if it complies with the necessary conditions for which it was designed, to that end the patient puts his shoes on and walks with the recently molded insole.

10. A process for producing plantar orthoses or insoles taking a footprint while walking and directly molding according to claims 8 and 9, the molding process in a weight-bearing standing position is **characterized in that**:
- A height-adjustable vacuum cushion with a connection to the vacuum melting machine is needed.
- The cushion consists of an enclosure completely adaptable to any shape.
- The patient, wearing the insulating cotton socks, is seated with his feet resting on the cushion adjusted to the height of the heel the patient normally uses and according to the diagnosis, and the patient stands up; new corrections can be made and when the supports are considered to be correct, a vacuum is performed on the cushion, the weight-bearing footprint remaining therein.
- Steps two, three, four and five of the molding in a seated position are repeated.
- The patient is placed on the footprint of the previous step, removing, the polystyrene bag and the insole before the complete cooling, and it is checked to see if it has stabilized.
- The process ends with the same steps seven and eight of the molding in a seated position.

11. A process for producing plantar orthoses or insoles taking a footprint while walking and directly molding according to claims 1 and 10, the height-adjustable vacuum cushion is **characterized in that** the cushion itself (1) is integrated by a cushioned body (2) internally filled with microspheres (3) and externally provided with a valve (5) for carrying out the internal vacuum, the body (2) being the height-adjustable portion of said cushion by means of a controlled lifting element (9), assembled on an assembly support platform (8), and acting in an articulation point (4), with an also cushioned fixed portion of the cushion (6) on which a vertical frame or harness (7) is arranged for the patient to hold on to.

12. A process for producing plantar orthoses or insoles taking a footprint while walking and directly molding according to claims 8, 9, 10 and 11, the molding combining both forms is **characterized in that** the first five steps of the molding in a seated position are followed.
- The patient stands up on the cushion adjusted to the height of the heel he usually uses and is kept in this position until the insole has cooled down, but the vacuum of the height-adjustable cushion is not carried out, the patient simply rests on the cushion until the insoles have cooled down.
- The process ends with the same steps seven and eight of the molding in a seated position.

13. A process for producing plantar orthoses or insoles taking a footprint while walking and directly molding according to claims 1 and 2, the process for finishing plantar orthoses is **characterized in that** references will be taken from steps one, two, there and four obtained in the walking process.
